# EUROPEAN PATENT SPECIFICATION

(11) **EP 1 363 614 B1**
(45) Date of publication and mention of the grant of the patent: **27.04.2005**
(21) Application number: 02710006.4
(22) Date of filing: 15.01.2002
(51) Int. Cl.: A61K 31/18, A61P 13/02, C07C 311/08

(54) **USE OF 2-AMINO-1-(4-HYDROXY-3-METHANESULPHONAMIDOPHENYL)ETHANOL FOR TREATING URINARY INCONTINENCE**
VERWENDUNG VON 2-AMINO-1-(4-HYDROXY-3-METHANESULPHONAMIDOPHENYL)ETHANOL ZUR BEHANDLUNG VON HARNINKONTINENZ
UTILISATION DE 2-AMINO-1-(4-HYDROXY-3-METHANESULPHONAMIDOPHENYL)ETHANOL POUR LE TRAITEMENT DE L'INCONTINENCE D'URINE

(30) Priority: 01.02.2001 DE 10104369
(43) Date of publication of application: 26.11.2003
(73) Proprietor: Boehringer Ingelheim Pharma GmbH & Co. KG, 55216 Ingelheim am Rhein (DE)
(72) Inventor: POUZET, Pascale, 88400 Biberach/Riss (DE); ESSER, Franz, 55218 Ingelheim am Rhein (DE); KITAGAWA, Hisato, Toyono-gun, Osaka 563-0104 (JP); ISHIGURO, Naoki, Toyonogun, Osaka 563-0104 (JP); MURAMATSU, Ikunobu, Yoshida-gun, Fukui 910-1134 (JP)
(86) International application number: PCT/EP2002/000311
(87) International publication number: WO 2002/060421

(56) References cited:
- EP-A- 0 538 469
- WO-A-96/32939
- WO-A-96/38143
- WO-A-97/06136
- US-A- 3 341 584
- LARSEN, AUBREY A. ET AL: "Sulfonanilides. II. Analogs of catecholamines" J. MED. CHEM. (1967), 10(3), 462-72 , XP002199471 cited in the application
- LECLERC G ET AL: "SYNTHESIS AND STRUCTURE ACTIVITY RELATIONSHIPS AMONG ALPHA ADRENERGIC RECEPTOR AGONISTS OF THE PHENYL ETHANOLAMINE TYPE" JOURNAL OF MEDICINAL CHEMISTRY, vol. 23, no. 7, 1980, pages 738-744, XP002199472 ISSN: 0022-2623
- ANDERSSON K E: "Drug therapy for urinary incontinence." BAILLIERE'S BEST PRACTICE & RESEARCH. CLINICAL OBSTETRICS & GYNAECOLOGY. ENGLAND APR 2000, vol. 14, no. 2, April 2000 (2000-04), pages 291-313, XP008003245 ISSN: 1521-6934
- HU BAIHUA ET AL: "(4-Piperidin-1-yl)phenyl amides: Potent and selective human beta3 agonists." JOURNAL OF MEDICINAL CHEMISTRY, vol. 44, no. 9, 26 April 2001 (2001-04-26), pages 1456-1466, XP002199473 ISSN: 0022-2623

## Description

The present invention relates to medicaments containing 2-amino-1-(4-hydroxy-3-mathanesulphonamidophenyl)ethanol, one of the two optical isomers thereof and/or the pharmacologically acceptable salts thereof, particularly for treating urinary incontinence.

The compound according to the invention has the following structure:
Formula I:

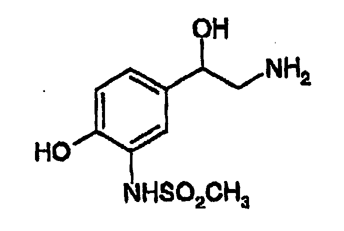

### Prior art:

By Incontinence is meant an involuntary release of urine, i.e. weakness of the bladder. The various manifestations of urinary incontinence include urge incontinence, reflex incontinence, overflow incontinence and stress incontinence. The most common form of urinary incontinence is stress incontinence. This affects women, in particular, after more or less difficult childbirth. The reason for this is that pregnancy and labour easily lead to a weakening of the pelvic floor. Other causes of incontinence may lie, for example, in damage to the nerves of the pelvic floor, a congenitally short urethra or damage to the sphincter muscle.

According to WO 96/32939 it is beneficial to use alpha-1L-agonists in the treatment of urinary incontinence, as they act selectively on the adrenoreceptors of the bladder and thus have a crucial effect on the tonicity of the urethra, without significantly affecting the cardiac circulatory system.

EP 0 538 469 describes 2-amino-1-(2-fluoro-5-methanesulphonamidophenyl)ethanol and the use thereof for treating urinary incontinence.

### Description of the invention

Surprisingly, it has now been found that the compound of formula I has an outstanding agonistic effect on alpha-1 L-receptors. The substance acts highly selectively on the bladder and inhibits urinary urgency.

The aim is therefore to develop a medicament with which urinary incontinence can be treated in a controlled manner.

A further objective is to develop medicaments which act selectively on the contractile mechanisms of the bladder without seriously affecting other organs such as e.g. peripheral blood vessels.

Another objective is to develop a medicament for treating urinary incontinence, in particular stress urinary incontinence, which has a relatively long-lasting effect.

Preferably, a drug which can be administered orally should be developed.

A further aim is to develop a non-toxic drug with few side-effects.

The overall aim of the present invention is therefore to find an active substance having the profiles described above and to develop a suitable medicament therefrom.

### Detailed description of the invention

The racemate of the compound according to the invention has been known since the 1960s.
It is described, for example, in US Patent 3,341,584 or GB 993,584. These publications mention that the racemate has a stimulant effect on alpha- and/or beta-receptors. The Journal of Medicinal Chemistry 10 (1967) page 467 describes an alpha-adrenergic effect.

The enantiomerically pure forms of the compound, i.e. the R- or S- form are not known as pharmaceutical substances. Also not known is the use of the compound, as a racemate or in enantiomerically pure form, i.e. in the R- or S-form, in a pharmaceutical composition for treating urinary incontinence, in particular stress urinary incontinence.

The compound is used in a pharmaceutical composition in the form of the racemate or one of the pure enantiomers.

The substance may be used both as a free base and as an acid addition salt.
Examples of such salts are salts of inorganic acids such as hydrochloric acid, hydrobromic acid, sulphuric acid, phosphoric acid or organic acids such as acetic acid, citric acid, tartaric acid, malic acid, succinic acid, fumaric acid, p-toluenesulphonic acid, benzenesulphonic acid, methanesulphonic acid, lactic acid, or ascorbic acid.
The hydrochloride is preferably used.

The compound according to the invention may be administered as a medicament by oral route, by inhalation, intranasally, intravenously, subcutaneously intramuscularily, transdermally, vaginally, rectally or as a suppository. Oral administration is preferred.

The compound may be administered on its own or in conjunction with other appropriate active substances.

To determine the optimum dose of the active substance for urinary incontinence, various parameters have to be taken into consideration, such as the age and body weight of the patient, and the nature and stage of the complaint.

The preferred dose for humans is between 0.001 mg and 1 g per day, preferably between 0.001 mg and 500 mg and most preferably between 0.01mg and 100 mg and most preferably between 0.01mg and 10 mg.

In some cases a smaller amount may be sufficient, whereas in other cases a larger total amount may be needed.

The total daily dose may be taken in one go or in several portions depending on the treatment regimen. The treatment regimen may also prescribe intervals of more than one day between doses of the drug.

The active substance according to the invention may be administered orally in various formulations, e.g. as a solid, in liquid form, as a powder, in the form of tablets, as a coated tablet, sugar-coated tablets, as an oral disintegrating tablet, as a sublingual tablet, in a capsule, in granulated form, as a suspension, solution, emulsion, elixir or syrup, in the form of drops or in other forms.

Capsules may be produced, starting from a powder of the kind mentioned above or other powders, which are packed into a capsule, preferably a gelatine capsule. It is also possible to introduce lubricants known from the prior art into the capsule or to use them to seal the two halves of the capsule. The dissolution rate of a capsule can be increased by the addition of disintegrant or solubilising substances, such as, for example, carboxymethylcellulose, carboxymethylcellulose calcium, lowly-substituted hydroxypropylcellulose, calcium carbonate, sodium carbonate, sodium carboxymethyl starch, crospovidone, croscarmellose sodium and other substances. The active substance may be contained in the capsule not only as a solid but also in solution or in suspension, e.g. in vegetable oil, polyethyleneglycol or glycerol, using surfactants.

Tablets (including vaginal tablets) may be prepared in which the powdered mixture is processed to form granules, mixed with other substances if necessary and then further compressed, for example. The tablets may contain various excipients, e.g. starches, lactose, sucrose, glucose, sodium chloride, urea for soluble or injectable tablets, amylose or various types of cellulose as described above.
Glycerol or starch may be added, for example, as moisture retaining agents.

Starch, alginic acid, calcium alginate, pectic acid, powdered agar-agar, formaldehyde gelatine, calcium carbonate, sodium bicarbonate, magnesium peroxide and amylose, for example, may be used as disintegrants.

Sucrose, stearin, solid paraffin (preferably with a melting point in the range from 50-52°C), cocoa butter and hydrogenated fats may be used as anti-disintegrants or solution retardants.

Suitable resorption accelerators include, *inter alia*, quaternary ammonium compounds, sodium lauryl sulphate, saponines.

Ether may be used as a binder distributor, for example, and cetylalcohol, glycerol monostearate, starch, lactose, wetting agents (e.g. Aerosol OT, Pluronics, Tweens) may be used as hydrophilisers or breakdown accelerators

Moreover, the following may be considered as tablet-making excipients in general: Aerosil, Aerosol OT ethylcellulose, Amberlite resin, XE-88, Amijel, Amisterol, amylose, Avicel microcrystalline-cellulose, bentonite, calcium sulphate, Carbowax 4000 and 6000, carrageenin, castor wax, cellulose, microcrystalline cellulose, dextrane, dextrin, base for pharmaceutical tablets, kaolin, spray dried lactose (USP), lactosil, magnesium stearate, mannitol, mannitol granular N. F. methylcellulose, Miglyol 812 neutral oil, powdered milk, lactose, nal-tab, Nepol-amylose, Pöfizer crystalline sorbitol, plasdone, polyethyleneglycols, polyvinylpyrrolidone, Précirol, calves foot oil (hydrogenated), base for melting tablets, silicones, stabiline, Sta-rx 1500, Syloid, Waldhof tablet base, Tablettol, talcum cetylatum and stearatum, Tego metal soaps, dextrose and tylose. The tabletting adjuvant K (M25) is particularly preferred and also meets the requirements of the following pharmacopoeias: DAB, Ph, Eur, BP and NF.

Other excipients from the prior art may also be used.

The tablets may be produced by direct compression.

Other formulations suitable for oral administration may also be prepared, such as suspensions, solutions, emulsions, syrups, elixirs, etc. If desired, the compound may be microencapsulated.

A powder may be prepared, for example, by reducing the particles of active substance to a suitable size by grinding.
Diluted powders may be prepared by finely grinding the powdered substance with a non-toxic carrier such as lactose and producing it as a powder. Other carrier materials which are suitable for this purpose are other carbohydrates such as starch or mannitol. If desired, these powders may contain flavourings, preservatives, dispersants, colourings and other pharmaceutically excipients.

The compound may be administered parenterally by dissolving, emulsifying or suspending it in a liquid and injecting it by subcutaneous, intramuscular or intravenous route. Suitable solvents include, for example, water or oily media.

To prepare suppositories the compound may be formulated with low-melting and water-soluble or water-insoluble materials such polyethyleneglycol, cocoa butter, higher esters (e.g. myristyl palmitate) or mixtures thereof.

### Examples

### 1. Metabolism

To determine the metabolism the enzyme CYP2D6 was allowed to act on the hydrochloride of the compound **1** according to the formula I (2-amino-1-(4-hydroxy-3-methanesulphonamidophenyl)ethanol). After 30 minutes a check was made to determine how much of the substance had been broken down by the enzyme. (-)-R-2-Amino-1-(2-fluoro-5-methanesulphonamido-phenyl)ethanol **2** was used as a comparison.

| Compound | % Substrate breakdown after 30 minutes' incubation with CYP2D6 |
|---|---|
| **1** | 1.4 |
| **2** | 2.1 |

### 2. Efficacy and selectivity

The efficacy and selectivity of the compound according to the invention is determined as follows, using the same findings as described in 1:

| Compound | activity in the dog | activity on human urethra | selectivity in the dog |
|---|---|---|---|
| **1** | 103 | 120 | 53 |
| **2** | 79 | 48 | 40 |

Maximum contraction in the dog and activity on human urethra are percentages of contraction compared with noradrenaline.

Selectivity in the dog is the difference obtained from the percentage contraction of the dog's femoral artery at 10⁻⁵ M and percentage contraction of the carotid artery in the dog at 10⁻⁵ M.

### 3. Pharmaceutical Composition

### Example A: Tablets

| | |
|---|---|
| 2-amino-1-(4-hydroxy-3-methanesulphonamidophenyl)ethanol | 1 mg |
| Lactose | 105 mg |
| Microcrystaline cellulose | 30 mg |
| Corn starch | 30 mg |
| Povidon | 5 mg |
| Sodium carboxymethyl starch | 5 mg |
| Colloidal silica | 3 mg |
| Magnesium stearate | 1 mg |
| Total | 180 mg |

Preparation: The active substance is mixed with some of the excipients and granulated in the usual way. The granules are sieved, combined with the remaining excipients and compressed into tablets weighing 180 mg.

### Example B: Ampoules

| | |
|---|---|
| 2-amino-1-(4-hydroxy-3-methanesulphonamidophenyl)ethanol | 1.0 mg |
| Sodium chloride | 18.0 mg |
| Sufficient water for injection to make up to | 2.0 ml |

Preparation: The active substance and sodium chloride are dissolved in water for injection and transferred into glass ampoules in an aseptic condition.

### Example C: Capsules

| | |
|---|---|
| 2-amino-1-(4-hydroxy-3-methanesulphonamidopheyl)ethanol | 1 mg |
| Lactose | 178 mg |
| Magnesium stearate | 1 mg |
| Total | 180 mg |

Preparation: The active substance is mixed with the excipients and filled into capsules as known in the state of the art.

### Preparation of the racemic compound:

The racemic compound can be prepared according to the procedures disclosed in GB 993,584 or US 3,341,584.

### Preparation of the enantiomeric pure forms

The pure enantiomers of the compound of the present invention can be obtained f.e. by transforming them into an diasteriomeric salt, f.e. with tartaric acid or others as mentioned above, followed by separating the two diastereomeric salts from each other via crystallisation and then liberating the pure enantiomer as the free base by adding a strong amino-base or an alkali-hydroxide.

Another way to obtain the pure enantiomers is purifying the racemate via HPLC by using a chiral column.
Still another way is to transform the racemic mixture into diastereomers, f.e. the diastereomeric salts as described above, to separate the two different diastereomers, diastereomeric salts respectively and then to liberate the pure enantiomer again.

All separation procedures as such are well known in the art.

## Claims

1. R-enantiomer of a pharmaceutical substance according to formula I 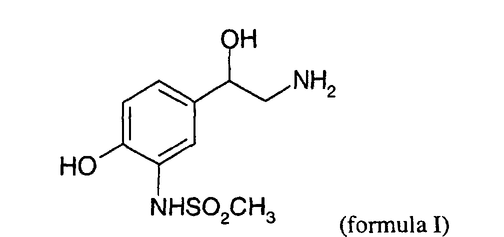 or a pharmacologically acceptable salt thereof for use as a medicament.

2. S-enantiomer of a pharmaceutical substance according to formula I 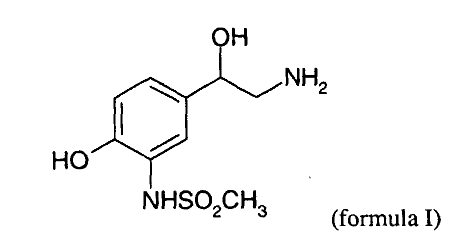 or a pharmacologically acceptable salt thereof for use as a medicament.

3. Pharmaceutical substance according to one of claims 1 or 2, **characterised in that** the pharmacologically acceptable salt is the hydrochloride.

4. Pharmaceutical composition containing a pharmaceutical substance according to one of claims 1 to 3.

5. Pharmaceutical composition containing a pharmaceutical substance according to one of claims 1 to 3 in an amount of between 0.001 mg and 1 g per day, preferably between 0.001 mg and 500 mg and most preferably between 0.01mg and 100 mg and most preferably between 0.01mg and 10 mg.

6. Pharmaceutical composition according to claim 4 or 5 in the form of a tablet, or a capsule.

7. Use of a pharmaceutical substance selected from a compound according to formula I 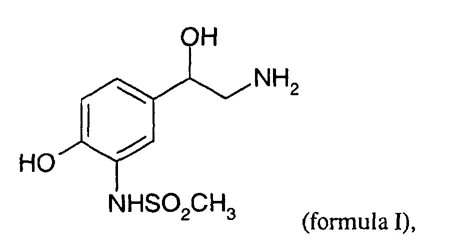 the R-enantiomer thereof, the S- enantiomer thereof, the racemate thereof or a pharmaceutically acceptable salt of any of them, preferably the hydrochloride or a pharmaceutical preparation comprising such a pharmaceutical substance for preparing a medicament for treating urinary incontinence, in particular stress urinary incontinence.

8. Use according to claim 7, **characterised in that** the pharmaceutical substance is only the R-enantiomer or a pharmaceutically acceptable salt thereof but not the S-form thereof.

9. Use according to claim 7, **characterised in that** the pharmaceutical substance is only S-enantiomer or a pharmaceutically acceptable salt thereof but not the R-form thereof.

10. Use according to claim 7, **characterised in that** the pharmaceutical substance is the racemate or a pharmaceutically acceptable salt thereof.

11. Use according to any of claims 7 to 10, **characterised in that** the pharmaceutical preparation contains said pharmaceutical substance in an amount of between 0.001 mg and 1 g per day, preferably between 0.001 mg and 500 mg and most preferably between 0.01mg and 100 mg and most preferably between 0.01mg and 10 mg.

12. Use according to any of claims 7 to 11, **characterised in that** the pharmaceutical substance or the pharmaceutical preparation is for oral administration.

13. Use according to claim 12, **characterised in that** the pharmaceutical substance or the pharmaceutical preparation is to be administered in the form of a tablet, or a capsule.

14. Use according to any of claims 7 to 10, **characterised in that** the pharmaceutical substance or the pharmaceutical preparation is for transdermal, parenteral, rectal or vaginal administration.

## Patentansprüche

1. R-Enantiomer einer pharmazeutischen Substanz der Formel I 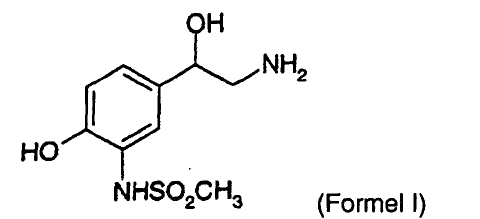 oder ein pharmakologisch annehmbares Salz davon zur Verwendung als Arzneimittel.

2. S-Enantiomer einer pharmazeutischen Substanz der Formel I 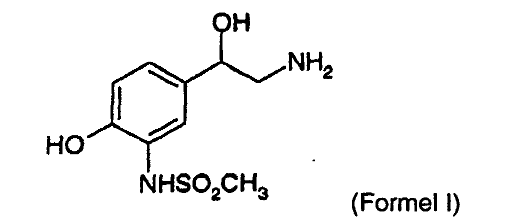 oder ein pharmakologisch annehmbares Salz davon zur Verwendung als Arzneimittel.

3. Pharmazeutische Substanz nach einer der Ansprüche 1 oder 2, **dadurch gekennzeichnet, dass** das pharmakologisch annehmbare Salz das Hydrochlorid ist.

4. Pharmazeutische Zusammensetzung enthaltend eine pharmazeutische Substanz nach einem der Ansprüche 1 bis 3.

5. Pharmazeutische Zusammensetzung enthaltend eine pharmazeutische Substanz nach einem der Ansprüche 1 bis 3 in einer Menge zwischen 0,001 mg und 1 g pro Tag, vorzugsweise zwischen 0,001 mg und 500 mg, besonders bevorzugt zwischen 0,01 mg und 100 mg und am meisten bevorzugt zwischen 0,01 mg und 10 mg.

6. Pharmazeutische Zusammensetzung nach Anspruch 4 oder 5 in Form einer Tablette oder einer Kapsel.

7. Verwendung einer pharmazeutischen Substanz ausgewählt aus einer Verbindung der Formel I 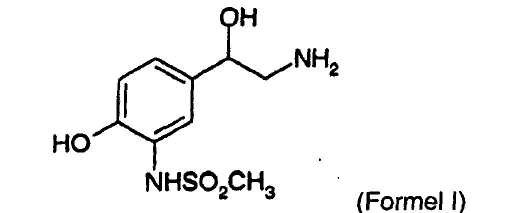 des R-Enantiomers davon, des S-Enantiomers davon, des Racemats davon oder eines pharmazeutisch annehmbaren Salzes von irgendeinem davon, vorzugsweise des Hydrochlorids, oder eines pharmazeutischen Präparates, das eine derartige pharmazeutische Substanz enthält, für die Herstellung eines Arzneimittels zur Behandlung von Harninkontinenz, insbesondere Stress-bedingter Harninkontinenz.

8. Verwendung nach Anspruch 7, **dadurch gekennzeichnet, dass** die pharmazeutische Substanz nur das R-Enantiomer oder ein pharmazeutisch annehmbares Salz davon, nicht aber die S-Form davon ist.

9. Verwendung nach Anspruch 7, **dadurch gekennzeichnet, dass** die pharmazeutische Substanz nur das S-Enantiomer oder ein pharmazeutisch annehmbares Salz davon, nicht aber die R-Form davon ist.

10. Verwendung nach Anspruch 7, **dadurch gekennzeichnet, dass** die pharmazeutische Substanz das Racemat oder ein pharmazeutisch annehmbares Salz davon ist.

11. Verwendung nach irgendeinem der Ansprüche 7 bis 10, **dadurch gekennzeichnet, dass** das pharmazeutische Präparat die pharmazeutische Substanz in einer Menge zwischen 0,001 mg und 1 g pro Tag, vorzugsweise zwischen 0,001 mg und 500 mg, besonders bevorzugt zwischen 0,01 mg und 100 mg und am meisten bevorzugt zwischen 0,01 mg und 10 mg enthält.

12. Verwendung nach irgendeinem der Ansprüche 7 bis 11, **dadurch gekennzeichnet, dass** die pharmazeutische Substanz oder das pharmazeutische Präparat für die orale Verabreichung ist.

13. Verwendung nach Anspruch 12, **dadurch gekennzeichnet, dass** die pharmazeutische Substanz oder das pharmazeutische Präparat in Form einer Tablette oder Kapsel zu verabreichen ist.

14. Verwendung nach irgendeinem der Ansprüche 7 bis 10, **dadurch gekennzeichnet, dass** die pharmazeutische Substanz oder das pharmazeutische Präparat für die transdermale, parenterale, rektale oder vaginale Applikation ist.

## Revendications

1. Enantiomère R d'une substance pharmaceutique selon la formule 1 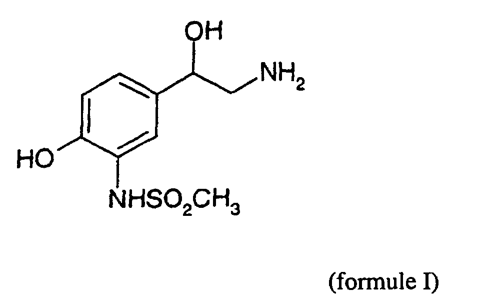 ou sel pharmacologiquement acceptable de celui-ci destiné à être utilisé comme médicament.

2. Enantiomère S d'une substance pharmaceutique selon la formule I 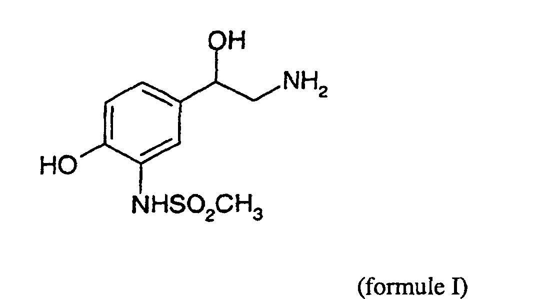 ou sel pharmacologiquement acceptable de celui-ci destiné à être utilisé comme médicament.

3. Substance pharmaceutique selon l'une des revendications 1 ou 2 **caractérisée en ce que** le sel pharmacologiquement acceptable est le chlorhydrate.

4. Composition pharmaceutique contenant une substance pharmaceutique selon l'une des revendications 1 à 3.

5. Composition pharmaceutique contenant une substance pharmaceutique selon l'une des revendications 1 à 3 en une quantité entre 0,001 mg et 1 g par jour, de préférence entre 0,001 mg et 500 mg et de manière particulièrement préférable entre 0,01 mg et 100 mg et de manière particulièrement préférable entre 0,01 mg et 10 mg.

6. Composition pharmaceutique selon la revendication 4 ou 5 sous forme d'un comprimé ou d'une capsule.

7. Utilisation d'une substance pharmaceutique choisie parmi un composé selon la formule I 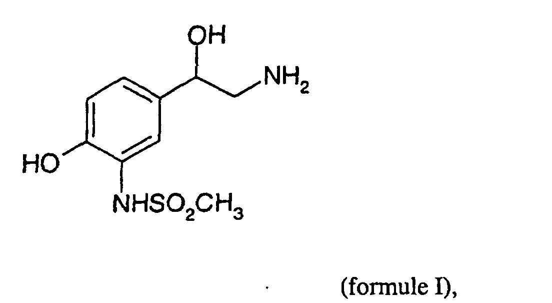 son énantiomère R, son énantiomère S, son racémate ou un sel pharmaceutiquement acceptable de l'un quelconque de ceux-ci, de préférence le chlorhydrate ou d'une préparation pharmaceutique comprenant une telle substance pharmaceutique pour préparer un médicament pour traiter l'incontinence urinaire, en particulier l'incontinence urinaire de stress.

8. Utilisation selon la revendication 7 **caractérisée en ce que** la substance pharmaceutique est seulement l'énantiomère R ou un sel pharmaceutiquement acceptable de celui-ci mais pas sa forme S.

9. Utilisation selon la revendication 7 **caractérisée en ce que** la substance pharmaceutique est seulement l'énantiomère S ou un sel pharmaceutiquement acceptable de celui-ci mais pas sa forme R.

10. Utilisation selon la revendication 7 **caractérisée en ce que** la substance pharmaceutique est le racémate ou un sel pharmaceutiquement acceptable de celui-ci.

11. Utilisation selon l'une quelconque des revendications 7 à 10 **caractérisée en ce que** la préparation pharmaceutique contient ladite substance pharmaceutique en une quantité entre 0,001 mg et 1 g par jour, de préférence entre 0,001 mg et 500 mg et de manière particulièrement préférable entre 0,01 mg et 100 mg et de manière particulièrement préférable entre 0,01 mg et 10 mg.

12. Utilisation selon l'une quelconque des revendications 7 à 11 **caractérisée en ce que** la substance pharmaceutique ou la préparation pharmaceutique est destinée à l'administration orale.

13. Utilisation selon la revendication 12 **caractérisée en ce que** la substance pharmaceutique ou la préparation pharmaceutique est destinée à être administrée sous forme d'un comprimé ou d'une capsule.

14. Utilisation selon l'une quelconque des revendications 7 à 10 **caractérisée en ce que** la substance pharmaceutique ou la préparation pharmaceutique est destinée à l'administration transdermique, parentérale, rectale ou vaginale.
